# EUROPÄISCHE PATENTANMELDUNG

(11) **EP 2 589 366 A1**
(43) Veröffentlichungstag der Anmeldung: **08.05.2013**
(21) Anmeldenummer: 11188099.3
(22) Anmeldetag: 07.11.2011
(51) Int. Cl.: A61J 1/03, B32B 5/02, B65D 75/32, A61K 9/00

(54) **Biologisch abbaubare Folienverpackung für orale Biologika**

(71) Anmelder: IDT Biologika GmbH, 06861 Dessau-Rosslau (DE)
(72) Erfinder: Wilke, Jürgen, 06366 Köthen (DE); Kaiser, Christian, 06046 Bernburg (DE); Schuster, Peter, 06846 Dessau-Roßlau (DE)
(74) Vertreter: Müller & Schubert

(57) **Zusammenfassung**

Die Erfindung betrifft einen versiegelbaren, biologisch abbaubaren Formkörper und dessen Herstellung sowie einen biologisch abbaubaren Folienverbund zur Bildung des Formkörpers und die Verwendung des Formkörpers zur Verpackung für orale Biologika, insbesondere oral applizierbare Impfstoffe.

Der Folienverbund besteht aus mindestens drei Schichten, wobei die Schichten jeweils mit einer Kaschierkleber-Schicht miteinander verbunden sind, eine der äußeren Schichten als Siegelschicht ausgebildet ist, die andere äußere Schicht aus einem Vliesstoff besteht und die mindestens eine mittlere Schicht eine Barriereschicht ist, wobei der Folienverbund biologisch abbaubar ist.

Aus dem Folienverbund wird durch Thermoverformung ein Formkörper gebildet, der das Biologikum oder den Impfstoff aufnimmt und dann versiegelt wird.

## Beschreibung

Die Erfindung betrifft einen versiegelbaren, biologisch abbaubaren Formkörper und dessen Herstellung sowie einen biologisch abbaubaren Folienverbund zur Bildung des Formkörpers und die Verwendung des Formkörpers zur Verpackung für orale Biologika, insbesondere oral applizierbare Impfstoffe.

Das Problem der oralen Verabreichung von biologisch oder veterinärmedizinisch wirksamen Mitteln (Biologika, insbesondere Impfstoffen) an freilebende oder in Gefangenschaft lebende Wildtiere, Heimtiere und Haustiere ist bisher noch nicht befriedigend gelöst worden. Ein besonderes Problem stellen dabei wirksame Mittel dar, welche in flüssiger Form vorliegen oder verabreicht werden müssen. Ein großer und wichtiger Anwendungsbereich ist hierbei die Impfung gegen Tollwut bei freilebenden Füchsen. Bisher werden hierfür Köder verwendet, welche den Impfstoff in einer herkömmlichen Blisterpackung umhüllt von einer festen Trägersubstanz enthalten, welcher ein Lockmittel beigefügt ist. Derartige Köder werden beispielsweise in der DE 36 11 122 A1 oder der US-A 4,861,586 beschrieben.

Die Blisterpackungen weisen jedoch große Nachteile auf. Zum einen ist es leicht möglich, diese von der festen Trägersubstanz zu trennen. Damit werden die Köder nicht mehr in der Art von den Zieltieren angenommen, wie dies für die orale Applikation des Impfstoffes erforderlich ist. Vielmehr separieren die Tiere die Blisterpackung und verschmähen diese dann.

Auch ist das Öffnen der Blisterpackung schwierig. Die Füchse müssen in die Packung hineinbeißen, um die Aluminiumfolie zu perforieren und um an den Inhalt zu gelangen. Der Inhalt wird dann über die Maulkavität aufgenommen. Dabei genügen im Allgemeinen schon geringe Aufnahmemengen, um eine wirksame Immunisierung zu bewerkstelligen. Jedoch beißen die Tiere nur dann in den Köder, wenn dieser unversehrt und die Blisterpackung nicht zu erkennen ist.

Derartige Köder sind hingegen für andere Wildtiere vollkommen ungeeignet, da diese ein anderes Fressverhalten zeigen. So untersuchen Waschbären die Nahrung sehr genau und verzehren diese in kleinen Stücken, die abgebissen werden. Aus anatomischen Gründen lassen Form und Aufbau der Maulhöhle die ganzheitliche Aufnahme des Köders nicht zu.

Es ist daher eine Aufgabe der vorliegenden Erfindung, eine Verpackung für die orale Applikation von Biologika für Tiere zu schaffen, welche die Nachteile des Standes der Technik überwindet.

Gegenstand der vorliegenden Erfindung ist ein Behälter in Form eines versiegelbaren Formkörpers, welcher aus einem Folienverbund gebildet wird, wobei der Folienverbund biologisch abbaubar ist, über hohe Barriereeigenschaften gegenüber Wasser, Sauerstoff und Kohlendioxid verfügt, tiefziehfähig und sterilisierbar ist und eine Haftungsschicht zu hydrophilen Stoffen aufweist.

Gegenstand der Erfindung ist also ein versiegelbarer Formkörper zur Verpackung für orale Biologika, insbesondere orale Impfstoffe, gebildet aus mindestens einem Folienverbund, wobei der Folienverbund, mindestens drei Schichten umfasst, wobei die Schichten jeweils mit einer Kaschierkleber-Schicht miteinander verbunden sind, eine der äußeren Schichten als Siegelschicht ausgebildet ist, die andere äußere Schicht aus einem Vliesstoff besteht und die mindestens eine mittlere Schicht eine Barriereschicht ist, und wobei der Folienverbund biologisch abbaubar ist

Ein weiterer Gegenstand der vorliegenden Erfindung ist ein Verfahren zur Herstellung eines erfindungsgemäßen versiegelbaren Formkörpers sowie dessen Versiegelung.

Gegenstand der vorliegenden Erfindung ist ferner der Folienverbund an sich, wobei der Folienverbund biologisch abbaubar ist, über hohe Barriereeigenschaften gegenüber Wasser, Sauerstoff und Kohlendioxid verfügt, tiefziehfähig und sterilisierbar ist und eine Haftungsschicht zu hydrophilen Stoffen aufweist.

Gegenstand der vorliegenden Erfindung ist ferner die Verwendung eines Folienverbundes zur Herstellung eines Formkörpers zur Verpackung für Biologika, wobei der Folienverbund biologisch abbaubar ist, über hohe Barriereeigenschaften gegenüber Wasser, Sauerstoff und Kohlendioxid verfügt, tiefziehfähig und sterilisierbar ist und eine Haftungsschicht zu hydrophilen Stoffen aufweist.

### Kurze Beschreibung der Zeichnungen

Fig. 1a und 1b zeigen Ausführungsbeispiele des erfindungsgemäßen Folienverbundes.
Fig. 2 zeigt die Anordnung des erfindungsgemäßen Folienverbundes beim Aufbau einer Ausführungsform eines erfindungsgemäßen versiegelbaren Formkörpers.
Fig. 3a und 3b zeigen eine weitere Ausführungsform eines erfindungsgemäßen versiegelbaren Formkörpers, die Anordnung des erfindungsgemäßen Folienverbundes bei der Bildung des Formkörpers und den Formkörper in versiegeltem Zustand.

Der der Erfindung zugrunde liegende Folienverbund, also der Folienverbund, aus dem der versiegelbare Formkörper herstellbar ist, besteht aus mindestens drei Schichten, wobei die Schichten jeweils mit einer Kaschierkleber-Schicht miteinander verbunden sind, eine der äußeren Schichten als Siegelschicht ausgebildet ist, die andere äußere Schicht aus einem Vliesstoff besteht und die mindestens eine mittlere Schicht eine Barriereschicht ist, wobei der Folienverbund biologisch abbaubar ist.

Ein bevorzugter Folienverbund ist derart ausgebildet, dass die Siegelschicht eine Dicke besitzt zwischen 10 bis 100 µm, bevorzugt 30 bis 70 µm und insbesondere bevorzugt 50 µm, die Kaschierkleber-Schicht eine Dicke besitzt zwischen 1 bis 10 µm, bevorzugt 2 bis 5 µm und insbesondere bevorzugt 3 µm, die äußere Schicht aus Vliesstoff eine Dicke besitzt zwischen 0,1 bis 1 mm, bevorzugt 0,2 bis 0,5 mm und insbesondere bevorzugt 0,3 mm und die mindestens eine Barriereschicht eine Dicke besitzt zwischen 5 bis 50 µm, bevorzugt 10 bis 30 µm und insbesondere bevorzugt 20 µm.

Ein weiterhin bevorzugter Folienverbund enthält 1 bis 5, bevorzugt 3 und insbesondere bevorzugt 2 Barriereschichten.

Erfindungsgemäß ist der Folienverbund derart ausgebildet, dass die eine Außenseite des Folienverbundes siegelfähig ausgebildet ist. Derartige siegelfähige Ausbildungen sind dem Fachmann bekannt. Diese Seite des Folienverbundes steht mit dem zu verpackenden Gut direkt in Kontakt. Dabei ist es vorgesehen, dass das Material der Seite, die mit dem zu verpackenden Gut direkt in Kontakt steht, derart ausgewählt ist, dass keine Interaktion mit dem zu verpackenden Gut auftritt. Es muss also ausgeschlossen sein, dass das verpackte Gut durch die Verpackung geschädigt oder in der Wirkung beeinträchtigt wird.

Unter dem Begriff "siegelfähig" wird im Zusammenhang mit der vorliegenden Erfindung aber auch jede Ausgestaltung der Außenseite des Folienverbundes verstanden, die geeignet ist, mit der Außenseite eines weiteren Folienverbundes verbunden zu werden. Diese Verbindung kann durch Haftung, Haftklebung oder Verkleben der jeweiligen Außenseiten miteinander bewirkt werden. Dabei können entsprechende an sich bekannte Kleber oder Haftmittel oder Adhäsionsmittel verwendet werden.

Des Weiteren weist der Folienverbund eine Barriereschicht auf. Diese Barriereschicht bewirkt, dass Medien aus der Umwelt wie Wasser, Wasserdampf, Luft, Sauerstoff, Kohlendioxid und andere in der Atmosphäre vorhandene Stoffe nicht in das Innere der aus dem Folienverbund gebildeten Verpackung an das verpackte Gut gelangen und das verpackte Gut schädigen. Die Barriereschicht bewirkt andererseits, dass Bestandteile des verpackten Gutes, insbesondere auch Wasser, das Innere des Blisters nicht verlassen können.

Erfindungsgemäß ist es ferner wesentlich, dass die zweite Außenseite des Folienverbundes aus einem Vliesstoff besteht oder auf die Außenseite ein Vliesstoff aufgebracht ist. Dieser Vliesstoff stellt die Oberfläche der gebildeten Verpackung dar, welche nicht mit dem zu verpackenden Gut in Berührung kommt.

Der gesamte Folienverbund ist erfindungsgemäß biologisch abbaubar. Diese Eigenschaft der biologischen Abbaubarkeit wird durch die Normen EN 13432 und ASTM D.6400-99 definiert. Die biologische Abbaubarkeit wird dadurch erreicht, dass die Siegelschicht, die Barriereschicht, die Kaschierkleber-Schicht(en) und die Schicht aus Vliesstoff aus einem nativen Biopolymer, aus einem biobasierten Polymer, einem erdölbasierten Polymer oder deren Mischungen bestehen. Bevorzugt ist es dabei, dass der Kaschierkleber ein biologisch abbaubarer Kleber, beispielsweise ein Polyurethankleber ist.

Dabei ist das native Biopolymer ausgewählt aus Cellulose, Cellulosederivaten, Stärke und Stärkederivaten, das biobasierte Polymer ist ausgewählt aus Polylactiden, Polyhydroxybutyraten, Thermoplasten auf Ligninbasis, Ethoxyacrylaten auf der Basis von Ölen, und das erdölbasierte Polymer ist ausgewählt aus Polyestern, Polyurethanen, Polyvinylalkoholen, Polybutylenadipatterephthalaten, Polybutylensuccinaten, Polycaprolactonen und Polyglycoliden. Weitere geeignete biologisch abbaubare Polymere sind im Stand der Technik bekannt, wie diese beispielsweise in DE 196 30 235 A1, DE 198 11 773 A1 und DE 198 11 226 A1 beschrieben sind.

Der auf der Außenseite des Folienverbundes vorhandene oder die Außenseite des Folienverbundes bildende Vliesstoff ist ebenfalls biologisch abbaubar. Entsprechende Vliesstoffe sind beispielsweise aus der DE 44 09 465 A1 bekannt. Der Vliesstoff enthält mindestens eine aus oder durch enthaltene und/oder fermentierte Stärke abbaubare Polymer/Copolymerfaser, ausgewählt aus natürlichen oder mit synthetischen Polymeren modifizierten natürlichen Polymeren und/oder synthetischen Polymerfasern, welche durch biologische Stärke, z. B. aus Kartoffeln, Rüben, Zuckerrohr, Mais, Weizen und dergleichen, modifiziert wurden, insbesondere solche aus Polyolefinen und/oder Polyester/Copolyestern, welche z. B. aus Stärke, fermentierten Stärkeprodukten oder über Bakterienstämme modifizierter Pflanzen, wie Kartoffeln, Raps etc. gewonnen werden können, auch in Kombination mit mehreren, auch verschiedenen Sorten biologisch abbaubarer Polymerfasern und/oder Naturfasern oder Copolyamiden und/oder biologischen Fasern wie Jute, Hanf, Flachs oder dergleichen und/oder abbaubaren Zuschlagstoffen und/oder abbaubaren Folien. Entsprechend dem jeweiligen Anwendungsgebiet kann das Vlies oder der Vliesstoff hydrophob oder hydrophil ausgestaltet sein.

Die Vliesfasern können aus einer Folie durch z. B. Fibrillieren hergestellt werden, aber auch aus gesponnenen Fasern bestehen, welche als Vlies oder Vliesstoff mechanisch, durch z. B. Vernadeln oder Wasserstrahlverfestigung (Spunlaceverfahren) oder thermisch oder durch Binder oder eine Mischung aller dieser Möglichkeiten verfestigt werden. Dabei können die Folien und auch die gesponnenen Fasern zuvor ein- oder mehrdimensional gereckt sein. Das Vlies oder der Vliesstoff kann dann auf einer Schicht aufgebracht werden, beispielsweise mittels eines Klebers. Bevorzugt ist es dabei, dass der Vliesstoff aus einem Vlies besteht, das eine Faserlänge von 1 bis 10 cm, bevorzugt 2 bis 8 cm und insbesondere bevorzugt 4 cm besitzt.

Zur Verbesserung der Eigenschaften des Folienverbundes ist vorgesehen, dass mindestens eine der Schichten des Folienverbundes als Barriereschicht ausgebildet ist. Neben der Eigenschaft der biologischen Abbaubarkeit, welche durch das Polymer an sich gegeben ist, werden der Barriereschicht Zuschlagstoffe beigegeben oder es werden metallische und/oder oxidische Stoffe auf der Barriereschicht aufgebracht, um die Barriereeigenschaften zu bewirken und/oder zu verbessern. Eine aus Polyvinylalkohol gebildete Barriereschicht ist beispielsweise aus der DE 196 32 799 A1 bekannt. Entsprechende Barriereschichten mit metallischen oder oxidischen Stoffen sind aus der DE 43 28 767 A1 bekannt. Besonders geeignet sind bei den Metallen Silber, Aluminium, Eisen und dergleichen, bei den oxidischen Verbindungen Aluminiumoxid, Ceroxid, Hafniumoxid, Magnesiumoxid, Siliziumdioxid, Siliziummonoxid, Tantaloxid, Titandioxid, Titan(3)oxid, Titanmonoxid, Yttriumoxid, Zirkonoxid, Zirkonmonoxid und dergleichen oder Mischungen davon. Besonders bevorzugt ist es, dass die Barriereschicht biologisch abbaubar ist und ein Cellulosederivat umfasst, welches metallisiert ist oder Siliziumdioxid und/oder Aluminiumoxid enthält.

Um die geforderten Gebrauchseigenschaften des Folienverbundes und des daraus hergestellten versiegelbaren oder versiegelten Formkörpers zu gewährleisten, ist es vorteilhaft, dass der Folienverbund eine Zugfestigkeit von mehr als 50 Newton, eine Weiterreißfähigkeit von mehr als 10 Newton, eine Verbundhaftung von mehr als 1,7 Newton, eine Durchlässigkeit von maximal 0,30 g/m²/24h Wasserdampf bei 23 °C und 50 % relativer Luftfeuchte, eine Durchlässigkeit von maximal 1,44 cm³/m²/24h Kohlendioxid und/oder eine Durchlässigkeit von maximal 0,65 cm³/m²/24h Sauerstoff besitzt.

Es ist in diesem Zusammenhang wichtig hervorzuheben, dass die Barriereeigenschaften des Folienverbundes in beiden Richtungen wirksam sein sollten. Es ist wichtig, das im Formkörper enthaltene Gut vor Umwelteinflüssen, also vor Einflüssen, die von außen einwirken, zu schützen. Es muss aber auch sichergestellt sein, dass das im Formkörper enthaltene Gut nicht aus dem Formkörper austritt.

Der beschriebene Folienverbund ist in besonderer Weise geeignet, einen versiegelbaren Formkörper auszubilden, der in dann versiegelter Form für die orale Applikation von Biologika vorgesehen ist.

Somit ist ein Gegenstand der vorliegenden Erfindung auch ein versiegelbarer Formkörper, der aus zwei Bahnen des hierin beschriebenen Folienverbundes gebildet wird. Dabei ist es erfindungsgemäß vorgesehen, dass der Folienverbund der einzelnen Bahnen identisch oder unterschiedlich aufgebaut ist. Dies bedeutet, dass die Abfolge der Schichten oder die Anzahl der Barriereschichten in dem jeweiligen Folienverbund andersartig ausgestaltet sein kann als in dem anderen Folienverbund. Jedenfalls muss mindestens ein Folienverbund thermoverformbar ausgestaltet sein, um den Wirkstoff darin einzubringen. Es kann erfindungsgemäß aber vorgesehen sein, beide Bahnen des jeweiligen Folienverbundes thermisch zu verformen, wobei auch hierbei der jeweilige Folienverbund zu dem anderen Folienverbund identisch oder verschieden sein kann. Beide Bahnen zu verformen wird dann vorteilhaft sein, wenn größere Mengen an Wirkstoffen eingebracht werden sollen.

Die Herstellung eines erfindungsgemäßen versiegelbaren Formkörpers erfolgt derart, dass man in einer Bahn des Folienverbundes durch thermische Verformung derart eine Aufnahme ausbildet, dass die äußere Schicht aus Vliesstoff nach außen weist, die zweite Bahn des gleichen oder eines modifizierten Folienverbundes derart in Kontakt mit der ersten Bahn des Folienverbundes bringt, dass die jeweiligen Siegelschichten aufeinander zuweisen, und man nach Befüllen des Formkörpers die beiden Bahnen des Folienverbundes durch Wärmeanwendung an den Kontaktbereichen miteinander versiegelt. Besonders bevorzugt ist es erfindungsgemäß, dass beide Bahnen des Folienverbundes durch thermische Verformung jeweils eine Aufnahme ausbilden.

Die Herstellung eines erfindungsgemäßen versiegelbaren Formkörpers kann aber auch derart erfolgen, dass der Folienverbund, aus welchem der Formkörper ausgebildet wird, keine Siegelschicht aufweist, welche thermisch versiegelbar ist. In diesem Fall wird der Formkörper wie zuvor beschrieben durch thermische Verformung ausgebildet und dann erst mit einer Haft-, Klebe- oder Adhäsionsschicht versehen und erst dann, ggf. nach dem Befüllen des Formkörpers mit dem zu verpackenden Gut, durch Aufbringen eines weiteren Folienverbundes verschlossen (versiegelt). Diese Verfahrenvariante kann vorteilhaft sein, wenn die Wärmeanwendung bei üblichen Siegelschichten zu einer Beeinträchtigung des zu verpackenden Gutes führen kann. Die Verwendung einer Haft-, Klebe- oder Adhäsionsschicht kann auch in diesem Falle auf den Bereich der eigentlichen Kontaktstellen begrenzt werden.

Auch ist es erfindungsgemäß vorgesehen, die Formkörper derart auszubilden, dass im Bereich der Siegelnaht ein Materialüberschuss einer der Bahnen erhalten bleibt. Hierzu werden beispielsweise Bahnen mit unterschiedlichen Breiten miteinander versiegelt. Der aus der Siegelnaht ragende Materialüberschuss kann dann mittels geeigneter Verfahren bedruckt werden. Der aus der Siegelnaht hervorragende Materialüberschuss ist vorzugsweise derart dimensioniert, dass dieser mit einem etwaig vorhandenen Aufdruck auch aus dem gesamten Köder, also dem mit dem Ködermaterial umhüllten Formkörper, herausragt und sichtbar ist.

Der erfindungsgemäße versiegelbare oder versiegelte Formkörper ist in besonderer Weise für die orale Applikation von Biologika geeignet.

Als Biologika werden biologisch oder veterinärmedizinisch wirksame Mittel verstanden, welche auf klassischem und auf biotechnologischem Weg hergestellt werden. Hierzu zählen insbesondere Humanarzneimittel, Tierarzneimittel, Kontrazeptiva und Impfstoffe in Form von Totimpfstoffen, Lebendimpfstoffen oder attenuierten Lebendimpfstoffen, hergestellt mit klassischen oder biotechnologischen Verfahren. Die entsprechend zubereiteten Wirkstoffe können neben den eigentlichen Wirkstoffen noch Zusatzstoffe, Hilfsstoffe und Adjuvantien enthalten, die physiologisch annehmbar sind, also selbst nicht schädigend wirken.

Unter dem Begriff "oral" oder "orale Applikation" wird im Sinne der Erfindung die Verabreichung von Stoffen über dem Mund bzw. das Maul (per os) verstanden. Dabei kann der Stoff an sich bereits in der Mund- oder Maulhöhle freigesetzt und/oder resorbiert werden. Orale Biologika im Sinne der Erfindung umfassen aber auch Zubereitungen, welche erst im Magen oder im Darmtrakt freigesetzt und/oder resorbiert werden.

Der Formkörper an sich ist aus biologisch abbaubaren Materialien hergestellt. Somit ist die Ausbringung der mit Wirkstoffen befüllten Formkörper in die Umwelt, auch in größeren Mengen, ökologisch unbedenklich. Die Prüfung auf die biologische Abbaubarkeit kann nach den Kriterien einschlägiger Normen erfolgen, wie EN 13432 oder ASTM D.6400-99.

Weiterhin sind die Formkörper sterilisierbar. In Abhängigkeit von dem zu applizierenden Wirkstoff muss die Zubereitung und/oder der gesamte befüllte und versiegelte Formkörper einer Sterilisation unterzogen werden. Die Sterilisation kann mit thermischen oder chemischen Verfahren oder durch energiereiche Bestrahlung erfolgen. Hiergegen muss das Material des Folienverbundes beständig sein.

Durch die Barriereschicht werden die Formkörper bzw. der Folienverbund nahezu undurchlässig für aus der Umgebung einwirkende Stoffe wie Wasser, Wasserdampf, Sauerstoff, Kohlendioxid und andere in der Atmosphäre enthaltenen Stoffe. Die im Formkörper vorhandenen Biologika sind häufig sehr empfindlich und können durch Einwirkung der genannten Stoffe geschädigt werden und die Wirksamkeit verlieren. Daher müssen die Biologika vor diesen Umwelteinflüssen geschützt sein.

Der Folienverbund ist so ausgebildet, dass die Polymerschicht, die mit den Biologika in Berührung kommt, mit diesen nicht wechselwirkt und insbesondere keine schädigende Wirkung ausübt.

Der an der Außenseite des Formkörpers vorgesehene Vliesstoff schließlich erfüllt verschiedene Aufgaben. Der Vliesstoff kann je nach Wahl der verwendeten Ausgangsmaterialien hydrophob, hydrophil, lipophob oder lipophil ausgestaltet werden. Ferner stellt der Vliesstoff eine vergrößerte wirksame Oberfläche für den Formkörper dar. Wird der Formkörper wegen der Einwirkung des Zieltieres geöffnet oder platzt dieser auf, so läuft die darin in flüssiger oder viskoser Form enthaltene Zubereitung nicht vollständig aus, sondern wird zu einem nicht unerheblichen Teil vom Vliesstoff aufgesogen. Dies bedeutet, dass die Zubereitung weiterhin im Kontaktbereich zum Zieltier verbleibt und damit die Wahrscheinlichkeit der oralen Aufnahme durch das Zieltier vergrößert wird. Diese Wirkung kann durch die entsprechende galenische Zubereitung verstärkt werden, wie der Erhöhung der Viskosität bei Flüssigkeiten.

Der Vliesstoff kann aber auch durch die entsprechende Ausgestaltung der Oberflächeneigenschaften geeignet sein, die Haftung anderer Materialien an der Oberfläche zu verbessern. So kann der Formkörper beispielsweise in einen Köder eingebracht werden, wobei das Ködermaterial beispielsweise ein Fett ist. Auch für andere Ködermaterialien wie Eiweiße, Kohlenhydrate und dergleichen können die Oberflächeneigenschaften entsprechend eingestellt oder ausgewählt werden, um die Haftung zu verbessern. Dadurch wird die Haftung des Ködermaterials an der Oberfläche des Formkörpers verstärkt und die Separation der beiden Komponenten des Köders ist erschwert.

Die Erfindung wird nun anhand der Figuren und der darin angeführten Ausführungsbeispiele näher erläutert:

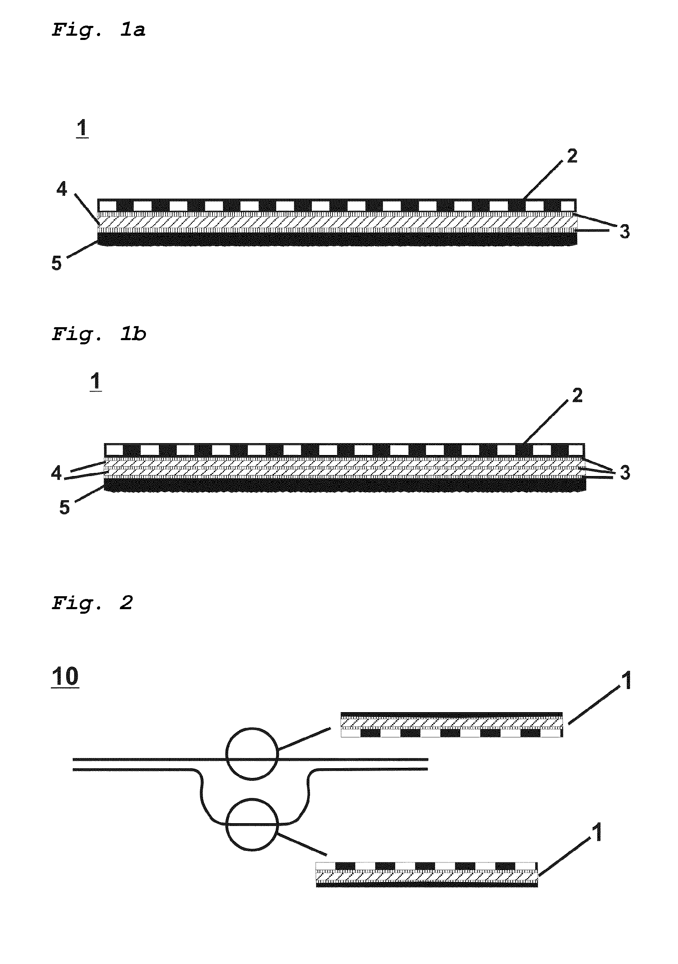

Die Figuren 1a und 1b zeigen Ausführungsbeispiele des erfindungsgemäßen Folienverbundes 1. Der in Figur 1a gezeigte erfindungsgemäße Folienverbund 1 setzt sich aus drei Schichten zusammen, die jeweils durch eine Kaschierkleber-Schicht 3 mit der benachbarten Schicht verbunden sind. Eine der beiden äußeren Schichten ist als Siegelschicht 2 ausgebildet. Die zweite äußere Schicht 5 ist aus Vliesstoff gebildet. Zwischen diesen Schichten befindet sich eine Barriereschicht 4.

In Figur 1b ist ein Ausführungsbeispiel des erfindungsgemäßen Folienverbundes 1 gezeigt, welcher mehr als eine Barriereschicht 4 besitzt. Gezeigt ist ein Folienverbund 1 mit zwei Barriereschichten 4, die durch Kaschierkleber-Schichten 3 miteinander verbunden sind. Eine der Barriereschichten ist durch eine Kaschierkleber-Schicht 3 mit Siegelschicht 2 verbunden, und eine der Barriereschichten 4 ist durch eine Kaschierkleber-Schicht 3 mit der äußeren Schicht 5, die aus Vliesstoff besteht, verbunden.

Figur 2 zeigt ein Ausführungsbeispiel eines erfindungsgemäßen versiegelbaren Formkörpers 10. Jeweils durch einen Kreis sind in dieser Figur die Bereiche gekennzeichnet, welche zusätzlich vergrößert dargestellt sind. Gebildet wird der Formkörper 10 durch zwei Bahnen des erfindungsgemäßen Folienverbundes 1. Der im unteren Bereich der Figur gezeigte Teil des Formkörpers 10, welcher durch eine Folienbahn gebildet wird, besitzt eine Vertiefung oder Aufnahme, wie sie durch Thermoverformung des Folienverbundes 1 erzeugt werden kann. Im oberen Bereich der Figur ist eine zweite Bahn des erfindungsgemäßen Folienverbundes 1 gezeigt. Beide Folienbahnen des erfindungsgemäßen Folienverbundes 1 sind derart zueinander ausgerichtet, dass jeweils die Siegelschichten 2 aufeinander zuweisen. In den Vergrößerungen ist der schematische Aufbau des Folienverbundes 1 gezeigt, wie er auch in Figur 1a gezeigt ist und im Rahmen dieser Figurenbeschreibung erläutert wird. Beispielhaft ist eine Ausführungsform des Folienverbundes mit einer Barriereschicht 4 gezeigt. Formkörper 10 kann jedoch auch durch jede andere Ausführungsform des erfindungsgemäßen Folienverbundes 1 gebildet werden.

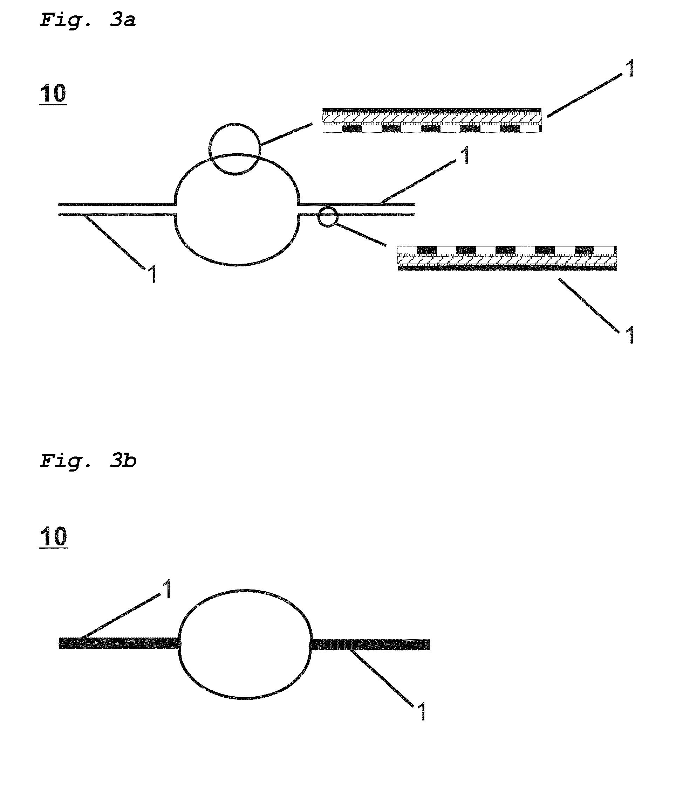

In Figuren 3a und 3b ist eine weitere Ausführungsform des erfindungsgemäßen versiegelbaren Formkörpers 10 gezeigt. Wie bei dem Ausführungsbeispiel der Figur 2 wird auch dieser Formkörper durch zwei Folienbahnen des erfindungsgemäßen Folienverbundes 1 gebildet. Jedoch wird in Figuren 3a und 3b eine Ausführungsform gezeigt, bei welcher beide Folienbahnen vor der Versiegelung verformt wurden. In beiden Folienbahnen ist eine Vertiefung bzw. Aufnahme ausgebildet. Gezeigt wird eine Ausführungsform, bei welcher beide Teile des versiegelbaren Formkörpers 10 identisch sind. In den vergrößerten Ausschnitten der Figur 3a ist dargestellt, welchen beispielhaften Aufbau die Folienbahnen aus dem erfindungsgemäßen Folienverbund 1 besitzen.

Figur 3a zeigt den versiegelbaren Formkörper 10, bei welchem die Bahnen des erfindungsgemäßen Folienverbundes 1 noch nicht in Kontakt miteinander gebracht wurden. Die Bahnen der Verbundfolie 1 sind bereits derart zueinander ausgerichtet, dass der Formkörper versiegelbar ist. In Figur 3b ist gezeigt, wie beide Folienbahnen des erfindungsgemäßen Folienverbundes 1 miteinander in Kontakt gebracht wurden, um den versiegelbaren Formkörper auszubilden. In der Praxis wird der Formkörper bei dieser Anordnung der Folienbahnen bereits befüllt sein und abschließend versiegelt werden.

### Beispiel

Ein erfindungsgemäßer Formkörper ist wie folgt ausgebildet:

Formkörper aus aufeinander gesiegelten, biologisch abbaubaren und tiefziehfähigen Folienverbunden, die von innen nach außen über den folgenden Schichtenaufbau verfügen:
● Siegelschicht aus biologisch abbaubarem Polyester
● Biologisch abbaubarer Zweikomponenten-Kleber mit der reaktiven Gruppe N-CO
● Barriereschicht aus biologisch abbaubarer metallisierter Celluloseschicht
● Biologisch abbaubarer Zweikomponenten-Kleber mit der reaktiven Gruppe N-CO
● Attraktivstoffhaftungsschicht aus biologisch abbaubarem Vliesstoff aus Viskose-Fasern

### Bezugszeichenliste

- 1: Folienverbund
- 2: Siegelschicht
- 3: Kaschierkleber-Schicht
- 4: Barriereschicht
- 5: Schicht aus Vliesstoff
- 10: versiegelbarer Formkörper

## Patentansprüche

1. Versiegelbarer Formkörper zur Verpackung für orale Biologika, insbesondere orale Impfstoffe, gebildet aus mindestens einem Folienverbund, wobei der Folienverbund, mindestens drei Schichten umfasst, wobei
die Schichten jeweils mit einer Kaschierkleber-Schicht miteinander verbunden sind,
eine der äußeren Schichten als Siegelschicht ausgebildet ist,
die andere äußere Schicht aus einem Vliesstoff besteht und
die mindestens eine mittlere Schicht eine Barriereschicht ist, und
wobei der Folienverbund biologisch abbaubar ist.

2. Versiegelbarer Formkörper, gemäß Anspruch 1, **dadurch gekennzeichnet, dass** die Siegelschicht, die Barriereschicht, die Kaschierkleber-Schicht und die Schicht aus Vliesstoff aus einem nativen Biopolymer, aus einem biobasierten Polymer, einem erdölbasierten Polymer oder deren Mischungen besteht.

3. Versiegelbarer Formkörper, gemäß Anspruch 2, **dadurch gekennzeichnet, dass** das native Biopolymer ausgewählt ist aus Cellulose, Cellulosederivaten, Stärke und Stärkederivaten, das biobasierte Polymer ausgewählt ist aus Polylactiden, Polyhydroxybutyraten, Thermoplasten auf Ligninbasis, Ethoxyacrylaten auf der Basis von Ölen, und das erdölbasierte Polymer ausgewählt ist aus Polyestern, Polyurethanen, Polyvinylalkoholen, Polybutylenadipatterephthalaten, Polybutylensuccinaten, Polycaprolactonen und Polyglycoliden.

4. Versiegelbarer Formkörper, gemäß Anspruch 2 oder 3, **dadurch gekennzeichnet, dass** das Polymer metallisiert ist.

5. Versiegelbarer Formkörper, gemäß einem der vorangehenden Ansprüche, **dadurch gekennzeichnet, dass** der Kaschierkleber ein biologisch abbaubarer Polyurethankleber ist.

6. Versiegelbarer Formkörper, gemäß einem der vorangehenden Ansprüche, **dadurch gekennzeichnet, dass** die Barriereschicht biologisch abbaubar ist und ein metallisiertes Cellulosederivat oder ein Siliziumdioxid und/oder Aluminiumoxid enthaltendes Cellulosederivat umfasst.

7. Versiegelbarer Formkörper, gemäß einem der vorangehenden Ansprüche, **dadurch gekennzeichnet, dass** der Vliesstoff aus einem Vlies besteht, das eine Faserlänge von 1 bis 10 cm, bevorzugt 2 bis 8 cm und insbesondere bevorzugt 4 cm besitzt.

8. Versiegelbarer Formkörper, gemäß einem der vorangehenden Ansprüche, **dadurch gekennzeichnet, dass** die Barriereschicht eine Sperrwirkung gegenüber den Fluiden Wasser, Luft, Sauerstoff und Kohlendioxid besitzt.

9. Versiegelbarer Formkörper, gemäß einem der vorangehenden Ansprüche, **dadurch gekennzeichnet, dass** die Siegelschicht eine Dicke besitzt zwischen 10 bis 100 µm, bevorzugt 30 bis 70 µm und insbesondere bevorzugt 50 µm,
die Kaschierkleber-Schicht eine Dicke besitzt zwischen 1 bis 10 µm, bevorzugt 2 bis 5 µm und insbesondere bevorzugt 3 µm,
die äußere Schicht aus Vliesstoff eine Dicke besitzt zwischen 0,1 bis 1 mm, bevorzugt 0,2 bis 0,5 mm und insbesondere bevorzugt 0,3 mm und
die mindestens eine Barriereschicht eine Dicke besitzt zwischen 5 bis 50 µm, bevorzugt 10 bis 30 µm und insbesondere bevorzugt 20 µm.

10. Versiegelbarer Formkörper, gemäß einem der vorangehenden Ansprüche, **dadurch gekennzeichnet, dass** der Folienverbund zwischen 1 bis 5, bevorzugt 3 und insbesondere bevorzugt 2 Barriereschichten enthält.

11. Versiegelbarer Formkörper, gemäß einem der vorangehenden Ansprüche, **dadurch gekennzeichnet, dass** der Folienverbund thermoverformbar ist.

12. Versiegelbarer Formkörper, gemäß einem der vorangehenden Ansprüche, **dadurch gekennzeichnet, dass** der Folienverbund eine Zugfestigkeit von mehr als 50 Newton, eine Weiterreißfähigkeit von mehr als 10 Newton, eine Verbundhaftung von mehr als 1,7 Newton, eine Durchlässigkeit von maximal 0,30 g/m²/24h Wasserdampf bei 23 °C und 50 % relativer Luftfeuchte, eine Durchlässigkeit von maximal 1,44 cm³/m²/24h Kohlendioxid und/oder eine Durchlässigkeit von maximal 0,65 cm³/m²/24h Sauerstoff besitzt.

13. Versiegelbarer Formkörper, gemäß einem der vorangehenden Ansprüche, **dadurch gekennzeichnet, dass** der Folienverbund in Form von Bahnen vorliegt und dass der Formkörper aus zwei Bahnen Folienverbund herstellbar ist.

14. Versiegelbarer Formkörper, gemäß Anspruch 13, **dadurch gekennzeichnet, dass** der Folienverbund der jeweiligen Bahnen unterschiedlich oder identisch ist, wobei mindestens ein Folienverbund thermoverformbar ist.

15. Verfahren zur Herstellung eines versiegelbaren Formkörpers gemäß Anspruch 13, wobei man in einer Bahn des Folienverbundes durch thermische Verformung derart eine Aufnahme ausbildet, dass die äußere Schicht aus Vliesstoff nach außen weist, die zweite Bahn des Folienverbundes gemäß einem der Ansprüche 1 bis 12 derart in Kontakt mit der ersten Bahn des Folienverbundes bringt, dass die jeweiligen Siegelschichten aufeinander zuweisen, und man nach Befüllen der Aufnahme des Formkörpers die beiden Bahnen des Folienverbundes durch Wärmeanwendung an den Kontaktbereichen miteinander versiegelt.
